(19) Europäisches Patentamt
European Patent Office
Office européen des brevets

(11) EP 0 739 373 B1

(12) EUROPEAN PATENT SPECIFICATION

(45) Date of publication and mention
of the grant of the patent:
**12.12.2001 Bulletin 2001/50**

(51) Int Cl.[7]: **C08J 5/02**, B29C 41/00,
A61L 31/00
// C08L75:04

(21) Application number: **94924366.1**

(22) Date of filing: **24.08.1994**

(86) International application number:
**PCT/GB94/01852**

(87) International publication number:
**WO 95/06082 (02.03.1995 Gazette 1995/10)**

(54) **ELASTOMERIC ARTICLES**

GUMMIGEGENSTÄNDE

ARTICLES ELASTOMERES

(84) Designated Contracting States:
**AT BE CH DE DK ES FR GB GR IE IT LI LU MC NL PT SE**

(30) Priority: **27.08.1993 GB 9317860**

(43) Date of publication of application:
**30.10.1996 Bulletin 1996/44**

(73) Proprietor: **Ansell Healthcare Products Inc.
Red Bank New Jersey 07701 (US)**

(72) Inventors:
• **GROMELSKI, Stanley, John
Canton, OH 44718 (US)**
• **NILE, Jeffery, Gordon
Alliance, OH 44601 (US)**
• **HARDWICK, Stephen, Thomas
York YO1 3NW (GB)**

• **MILNER, Richard
Selby York YO8 8EP (GB)**

(74) Representative: **Bauer, Robert, Dipl.-Ing. et al
Patentanwälte Boeters & Bauer
Bereiteranger 15
81541 München (DE)**

(56) References cited:
**EP-A- 0 257 225          WO-A-89/08672**

• **ELASTOMERICS, vol.110, no.8, 31 August 1978,
ISSN:0146-0706 pages 17 - 20 SADOWSKI J.S. ET
AL. 'Polyurethane Latexes for Coagulation
Dipping' cited in the application**
• **DATABASE WPI Week 9326, Derwent
Publications Ltd., London, GB; AN 93-208895 &
JP,A,5 132 567 (DAINIPPON INK & CHEM KK) 28
May 1993**

**Description**

The present invention relates to a method of manufacturing a thin-walled polyurethane article according to claim 1, articles prepared according to this method, and the use of a corresponding polyurethane emulsion.

Elastomeric thin-walled articles, such as gloves for surgical use are generally manufactured from natural rubber, usually by dipping a glove former into a vessel containing natural rubber latex. However, recently problems have been encountered with natural rubber surgical gloves since small quantities of proteins may leach from the glove into an open surgical site and in some instances cause an allergenic reaction to the patient.

Attempts have been made to manufacutre surgical gloves from synthetic materials such ass polyurethane. US Patent No. 4463156 describes, in Example 14, the manufacture of a mixed diol, 4,4'-diphenylmethane diisocyanate polyurethane. A 20% solids solution of the polyurethane was prepared and a glove produced by dipping a glove former into the suspension. However such polyurethane suspensions suffer from the disadvantage that they tend to be fairly unstable on storage and therefore do not lend themselves to production processes.

European Patent Publication No. 0413467 discloses polyurethane condoms which are manufactured by dipping a suitably shaped former into an organic solvent solution of a polyurethane. However, such processes have a major disadvantage in that undesirable solvents must be used thus giving rise to increased costs and problems with waste products.

Sadowski et al (J S Sadowski, B Martin and D D Gerst, "Polyurethane Latexes for Coagulation Dipping", Elastomerics, August 1978, 17-20) describes that polyurethane gloves may be made by the "Anode" process, that is, by dipping a former into a coagulant bath, followed by dipping into a polyurethane latex. The polyurethane in the latex was of a particle size in the range of from 0.06 to 0.3 μm. The latex also comprised a non-ionic surfactant. However, although Sadowski, in 1978, alleged that suitable gloves could be formed, prior to the present invention, polyurethane gloves manufactured by coagulation dipping have never been made commercially available. Thus there has been a long felt want for a commercially viable process for the emulsion dipping of polyurethane articles and polyurethane gloves in particular.

We have now surprisingly found that certain polyurethanes with a larger particle size than that disclosed by Sadowski can advantageously be formed into aqueous emulsion which are sufficiently unstable to electrolytes to permit thin layer coatings to be formed, e.g. in the manufacture of gloves, whilst the emulsions possess adequate shelf life if a plasticiser is added to the emulsions.

Thus, according to claim 1, we provide a method of manufacturing a thin-walled polyurethane article having a wall thickness in the range from 0.03 mm to 1.0 mm, the method comprising dipping a shaped former into an aqueous polyurethane emulsion comprising at least 50% w/w of particles with a mean particle size of greater than 1.0 μm, as measured e.g. by electron microscopy, in combination with a plasticiser.

The result obtained in this way is, in fact, surprising since plasticisers, which are high-boiling solvents or softening agents, have previously been added to a polymer only to facilitate processing or to increase flexibility or toughness.

By the term "mean particle size" is meant the mean diameter for generally spherical particles. The mean particle size of polyurethane emulsions can be measured using conventional methods known per se, such as electron microscopy or a conventional particle size analyser, e.g. a Malvern Analyser. Preferably, the mean particle size is from 1.0 μm to 2.5 μm, more preferably from 1.0 μm to 2.0 μm, and most preferably from 1.0 μm to 2.0 μm.

Preferably at least 50% w/w, more preferably at least 70% w/w, still more preferably at least 80% w/w and especially at least 90% w/w of the particles have a particle size of greater than 1.0 μm when measured using electron microscopy.

The claims 13 to 19 pertain to various thin-walled polyurethane articles prepared according to the method of claim 1. These articles, in spite of the relatively large particle size employed in their manufacturing, show a high degree of homogeneity and, accordingly, of tightness and tensible strength.

Claim 20 pertains to the use of an aqueous polyurethane emulsion as specified in claim 1 in the manufacture of a thin-walled polyurethane article.

We have found that the presence of a surfactant in the polyurethane emulsion increases the stability of a large particle size polyurethane emulsion and provides advantageous film forming properties.

Thus according to the invention we provide a method as hereinbefore described wherein the polyurethane emulsion comprises from 0.1 to 10% w/w of surfactant, preferably from 2 to 8% w/w, more preferably from 4 to 6% w/w, and most preferably approximately 5% w/w.

The amount of surfactant may vary according to the type of surfactant used, the nature of the polyurethane, the particle size of the polyurethane, the solids content of the emulsion, etc.. Generally however, the amount of surfactant present should be at least sufficient to exceed the critical micelle concentration in the emulsion. More particularly, it is preferred to use an amount of surfactant in the emulsion so that the surface tension of the emulsion, measured using conventional methods known per se, approaches that of natural rubber latex. Thus, it is preferred that the emulsion possesses a surface tension of from 20 to 50 dynes cm$^{-1}$, preferably from 25 to 45 dynes cm$^{-1}$, more preferably from 30 to 40 dynes cm$^{-1}$, and most preferably approximately 35 dynes cm$^{-1}$.

Any conventional surfactant may be used including non-ionic surfactants, ionic surfactants, e.g. anionic surfactants, or a mixture of two or more ionic and non-ionic surfactants. Of the ionic surfactants, anionic surfactants are preferred. Examples of suitable anionic surfactants include carboxylate surfactants. Carboxylate surfactants are typically derived from C 10 to C 20 straight chain fatty acids which may optionally be saturated or unsaturated. Conventionally known salts of carboxylate surfactants may be used, such salts including ammonium or alkyl ammonium salts, e.g. triethylamine or morpholine salts; or alkali metal salts, e.g. sodium or potassium salts. In addition to the fatty acids, salts of other acids may be used including oleic, ricinoleic and rosin acids or the n-alkyl sarcosides. The invention is not limited to the surfactants hereinbefore described and may also include mixtures of two or more surfactants.

Non-ionic surfactants are particularly preferred and examples of suitable non-ionic surfactants include those described in "Surfactant and Interfacial Phenomens" Miltan J Rosen, (John Wiley & Sons Inc (1978)). Examples of non-ionic surfactants which may be mentioned are, polyoxyethylene alkylphenols; alkylphenol ethoxylates, examples of alkylphenols are p-nonylphenol, p-octylphenol or p-dodecylphenol; polyoxyethylenated straight chain alcohols; alcohol ethoxylates (the alcohols are typically derived from coconut or tallow oils or are synthetic long carbon chain alcohols); polyoxyethylenated polyoxypropylene glycols (derived from ethylene oxide and propylene oxide); polyoxyethylenated mercaptans; long chain carboxylic acid esters, eg. glycerol and polyglyceryl esters of natural fatty acids, propylene glycol, sorbitol and polyoxyethylenated sorbitol esters; polyoxyethylene glycol esters and polyoxyethylenated fatty acids; alkanolamine condensates; alkanolamides, eg. alkanolamine/fatty acid condensates; and tertiary acetylenic glycols.

We have found that the use of a surfactant in the polyurethane results in residual surfactant being present in the thin walled article. Thus according to the invention we provide a thin walled polyurethane article comprising from 0.1 to 10% w/w of surfactant.

The amount of plasticiser present in the polyurethane emulsion may be from 0.1 to 40% w/w, preferably from 0.1 to 20% w/w more preferably from 0.1 to 10 w/w, and most preferably from 5 for 10% w/w plasticiser. Plasticisers which may be mentioned include the esters of diacids, such as esters of alkanedioic acids and especially esters of dibenzoic acid. The dipropylene glycol esters are preferred. Other plasticisers include polyadipates, such as polyneopentyl glycol adipate sold as DIOLPATE 160 (Trade Mark) by Macpherson Polymer.

The concentration of the polyurethane in the aqueous emulsion, ie. the solids content, may be from 10 to 60% w/w, preferably 20 to 60% w/w, more preferably 40 to 60% w/w, still more preferably 45 to 55% w/w, and most preferably 52 to 53% w/w.

The molecular weight of the polyurethane in the emulsion may vary, but preferably the weight average molecular weight ($M_w$) is from 50,000 to 1,000,000, more preferably from 50,000 to 800,000. The number average molecular weight ($M_n$) may be from 4000 to 1500, preferably from 5000 to 12000. Emulsions are especially preferred wherein the molecular weight distribution tends to be binodal, that is, the emulsion comprises a relatively high molecular weight component and a relatively low molecular weight component. The relatively high molecular weight component may have a weight average molecular weight in the region of from 50,000 to 150,000, preferably from 75,000 to 125,000, and most preferably approximately 100,000, whilst the relatively low molecular weight component may have a weight average in the region of from 1000 to 2000, preferably from 1200 to 1800, and most preferably approximately 1600. The relative ratio of the high molecular weight component to the low molecular weight component may be from 4:1 to 3:2, preferably 3:1.

According to the invention we also provide a thin walled polyurethane article wherein the polyurethane has a weight average molecular weight of from 50,000 to 1,000,000.

In addition the polyurethane emulsion may comprise a salt forming moiety to confer dispersibility, such as an amine, eg. diethanolamine or morpholine, or a cationic species, eg. sodium, potassium, ammonium, etc. Preferred amines include tertiary amines and especially tertiary alkyl amines, such as triethylamine.

We further provide the use of an aqueous polyurethane emulsion as hereinbefore described in the manufacture of a thin walled polyurethane article according to the invention.

A variety of polyurethanes may be used in the manufacture of the articles of the invention. However, preferred polyurethanes are anionic polyurethanes and especially those prepared from polyethers or polyesters. Aromatic polyesters may be used but aliphatic polyesters are preferred. Similarly, although aromatic diisocyanates may be used, aliphatic diisocyanates are preferred. It is especially preferred to use diisocyanates which can render improved flexibility to the polyurethane, such as cyclohexane diisocyanates and alkylcyclohexane diisocyanates. Preferred polyurethanes are those sold under the name UCECOAT (Trade Mark), such as UCECOAT XE102. The most preferred polyurethanes are those sold under the name WITCOBOND (Trade Mark) available from Baxenden in the UK. WITCOBOND 740 is especially preferred.

Thus according to the invention we provide a thin walled article made from a polyurethane selected from WITCOBOND and UCECOAT, especially WITCOBOND, eg. WITCOBOND 740.

The method according to the invention may advantageously include a coagulant dipping step in the process. Thus according to the invention we provide a method of manufacturing a thin walled polyurethane article which comprises first dipping a suitably shaped former into a coagulant solution, optionally drying the coagulant coated former, followed by dipping the coagulant coated former into a polyurethane emulsion as hereinbefore described.

Preferred coagulants are ionic coagulants, eg. mono, di- and tri- valent cations. Examples of monovalent cationic coagulants include quarternary ammonium salts such as cyclohexylamine salts, eg. cyclohexylamine acetate. Divalent cationic coagulants are preferred, such as alkaline earth metal salts, eg. calcium salts, or transition metal salts, eg. zinc salts. The nitrates of these salts are preferred and thus the most preferred coagulant is calcium nitrate. In addition to conventional coagulants, heat sensitising agents may also be used. Thus heat sensitising agents such as polyether siloxanes may be used, such agents are sold as coagulant WS by Bayer. Heat sensitivity coagulants are generally mixed with the polyurethane emulsion. The amount of coagulant present in the coagulant solution may be from 5 to 60% w/w solution, preferably from 5 to 30% w/w, more preferably from 5 to 20% w/w, still more preferably 8 to 12% w/w and most preferably approximately 10% w/w.

The method according to the invention may also include a curing step. Thus following dipping an appropriately shaped former into the polyurethane emulsion the formed article may be cured at from 60° to 150°C, preferably from 60° to 120°C and more preferably from 70° to 100°C, and most preferably at approximately 90°C. Curing times may vary according to, inter alia, the thickness of the formed article. Curing times of from 5 to 60 minutes are appropriate.

In addition, the method according to the invention may include a leaching step. Leaching may be carried out before or after curing the formed article, or leaching may be carried out both before and after curing.

The leaching process is intended to, inter alia, remove residual coagulant and/or surfactant. It may, however, be a characteristic of the thin-walled polyurethane articles according to the invention that residual coagulant and/or surfactant remains in the polyurethane. Thus the polyurethane article may comprise a coagulant residue of from 2 to 15mg/g of polyurethane, preferably from 2 to 12mg/g, and most preferably from 3 to 10mg/g.

In the case of a calcium nitrate coagulant the residual coagulant may be detectable in the form of calcium ions. Thus conventional detecting techniques known per se may be used in, eg. calcium ion analysis. Such methods include, inter alia, atomic absorption spectrometry.

In the formed article the polyurethane may comprise from 0.1 to 40% w/w of plasticiser residue, preferably from 0.1 to 20% w/w.

Additives may be included in the coagulant solution or the emulsion to improve the release properties of the polyurethane article allowing the cured article to be more easily removed from the former. The stripping of the article from the former may be improved by the inclusion of a detackifier. Any conventional detackifiers may be used including powders such as chalk, starch, eg. maize starch or corn starch, slays, lycopodium powders, talc or ground whiting. In addition liquid detackifiers conventionally used with natural rubbers may be used, these include silicone emulsions, silicone oils or polypropylene glycols.

The thin walled articles manufactured by the process of the invention may also be detackified by surface treatment, such as chlorination or bromination or surface coating. Powdered detackifiers may be applied in the coagulant or after the article has been cured, but the preferred detackifiers are carbonates, such as alkaline earth metal carbonates, eg. calcium carbonate. The level of detackifier present in the glove may vary, but preferred levels are from 0 1 to 10% w/w, preferably from 2.5 to 7.5% w/w, and most preferably approximately 5% w/w.

The thickness of the articles may be from 0.03 to 1.0mm, preferably from 0.05 to 0.8mm and most preferably from 0.05 to 0.5mm.

Such articles are advantageous in that the tensile strength or the force required to break the polyurethane remains sufficient to give the article utility. Thus the polyurethane article may have a tensile strength of from 2 MPa to 60 MPa, preferably from 5 to 40 MPa, more preferably from 10 to 40 MPa still more preferably from 15 to 40 MPa, and most preferably from 24 to 40 MPa.

In addition, the articles according to the invention have an advantageous value for elongation at break. Thus, an article according to the invention may have an elongation at break of greater than 450%, preferably from 500 to 600%, and most preferably approximately of 550%.

The thin walled articles of the invention may include gloves, eg. gloves for surgical use, condoms, sleeves, catheters, boots, bladders, balloons, teats, enema tips and other tubular articles.

In the following a number of comparative examples will be described relating to the invention but not providing the addition of a plasticiser:

Example 1

Preparation of a Sleeve

A glazed ceramic former in the shape of a flat plate was preheated in an oven at 90°C. The former was removed from the oven and dipped into a 20 w/w% solution of calcium nitrate hex-hydrate in industrial methylated spirits which also had dispersed within it 5 w/w% (based on solution) calcium carbonate powder.

The former was removed and then allowed to air dry for 30 seconds before being dipped into an approximately 50 w/w% solids emulsion of WITCOBOND 740 in purified water. The former was allowed to dwell for 5 seconds in the emulsion then withdrawn and allowed to air dry for 1 minute.

A polyurethane gel formed and the coated former were immersed in purified water at 70° for 2 minutes, withdrawn and then placed into an oven at 90°C for 30 minutes. The former was removed from the oven and allowed to cool before the polyurethane film was removed.

Example 2

Tensile Test

A 4mm wide dumbbell cutter was used to cut a sample of film. Reflective markers stuck to the parallel part of the test piece identified a 25mm length. A tensile test was carried out with a crosshead speed of 500mm min$^{-1}$ using a Zwick 1435 tensile testing machine. An extensometer measured the true strains during the test by following the reflective markers. The stresses at 100, 300 and 500% strain were recorded along with the stress and strain at break.

Table I

| Batch | Thickness | Stress at (MPa) | | | | Strain at |
|---|---|---|---|---|---|---|
| No. | (mm) | 100% | 300% | 500% | Break | Break (%) |
| | | | | | | |
| 1 | 0.170 | 2.08 | 4.05 | 23.1 | 42.0 | 553 |
| | (0.011) | (0.13) | (0.25) | (2.2) | (7.3) | (12) |
| 2 | 0.181 | 2.17 | 4.30 | 24.6 | 37.7 | 538 |
| | (0.029) | (0,03) | (0.03) | (1.5) | (4.4) | (8) |
| 3 | 0.213 | 2.01 | 3.96 | 21.2 | 34.5 | 544 |
| | (0.024) | (0.06) | (0.20) | (1.9) | (9.5) | (26) |
| 4 | 0.188 | 2.16 | 4.19 | 23.3 | 40.1 | 547 |
| | (0.027) | (0.24) | (0.45) | (2.6) | (4.8) | (8) |

Averages of six samples are shown within the standard deviation in brackets.

Example 3

Tensile Set

Set is defined as the permanent deformation following the application and removal of strain. A 4mm wide dumbbell cutter was used to cut a sample of film, and reflective markers were stuck on about 25mm apart. The sample was stretched to 300%, and immediately relaxed. The second gauge length between the reflective markers was measured under a very small load. Percentage set is defined as:

$$\% \text{ set} = \frac{(\text{2nd gauge length - 1st gauge length}) * 100\%}{(\text{1st gauge length})}$$

Table II

| Batch No. | Average | & Unrecovered No. of Tests s.d$_{(n-1)}$0.8 | Individual Results |
|---|---|---|---|
| NR latex | 6.7 | | |
| 1 | 15.2 | 3 | 14.9, 15.5, 15.1 |
| 2 | 15.9 | 3 | 15.5, 15.5, 16.6 |

Table II (continued)

| Batch No. | Average | & Unrecovered No. of Tests s.d$_{(n-1)}$0.8 | Individual Results |
|-----------|---------|-----------------------------------------------|--------------------|
| 3 | 11.7 | 3 | 16.4, 8.8, 9.8 |
| 4 | 14.2 | 3 | 10.9, 15.3, 16.3 |

Example 4

Preparation of a Glove

A hand shaped glazed former was preheated in an oven set at 90°C, the former was then removed from the oven and dipped into a 10% w/w solution of calcium nitrate tetrahydrate in industrial methylated spirits which also had dispersed within it 5% w/w (based on solution) calcium carbonate powder.

The former was removed and then allowed to air dry for 50 seconds before being dipped into an approx 53% w/w solids solution of WITCOBOND 740 in distilled water. The former was allowed to dwell for 0.1 seconds before being withdrawn and allowed to dry for 110 seconds.

The polyurethane gel formed and the former were immersed in distilled water at 50°C for 2 minutes, withdrawn and then placed into an oven at 90°C for 40 minutes. The former was then removed from the oven and allowed to cool before the glove was stripped.

Example 5

Preparation of a Condom

A penile shaped glass former was preheated in an oven set at 80°C. On removal from the oven the former was dipped into an approximately 55% w/w solids solution of WITCOBOND 740 in distilled water. The former was then slowly withdrawn in order to reduce the risk of loose excess material collecting at the end of the former. On withdrawal the former was rotated in order to eliminate the formation of a thick deposition at the tip of the condom. The thin gel on the former on withdrawal was allowed to dry for up to 1 minute before oven drying. The coated former was then placed in an oven kept at 80°C for 10 minutes. A second polyurethane coat was then applied by following a similar procedure to that hereinbefore described. The double coated former was then placed in another oven and kept at 80°C for 20 minutes. The condom was then stripped and treated with a detackifier (corn starch).

Example 6

Molecular Weight Determination

Molecular weight of the polyurethane was analysed using EASICAL (Trade Mark) polystyrene standards dissolved in tetrahydrofuran (2ml, 0.2%) by allowing the standard mix stick to stand in tetrahydrofuran for 30 minutes. The polyurethane samples were prepared using 0.03g of polyurethane dissolved in tetrahydrofuran (10ml) SOP/AD/170/246/262.

The samples were analysed using gel permeation chromatography (GPC) and a refractive index detector.

GPC Conditions

Column :         Polymer Labs Gel 10μm mixed bed and guard (from Phenomenex in the UK)
Temp :           35° C
Mobile Phase :   Tetrahydrofuran
Flow Rate :      1.0 ml/min

| Standard 1 | | Standard 2 | |
|---|---|---|---|
| | | | |
| 1: | 380 000 | 1: | 156 000 |
| 2: | 96 000 | 2: | 49 900 |
| 3: | 22 000 | 3: | 11 600 |
| 4: | 5 050 | 4: | 2 950 |
| 5: | 1 320 | 5: | 580 |

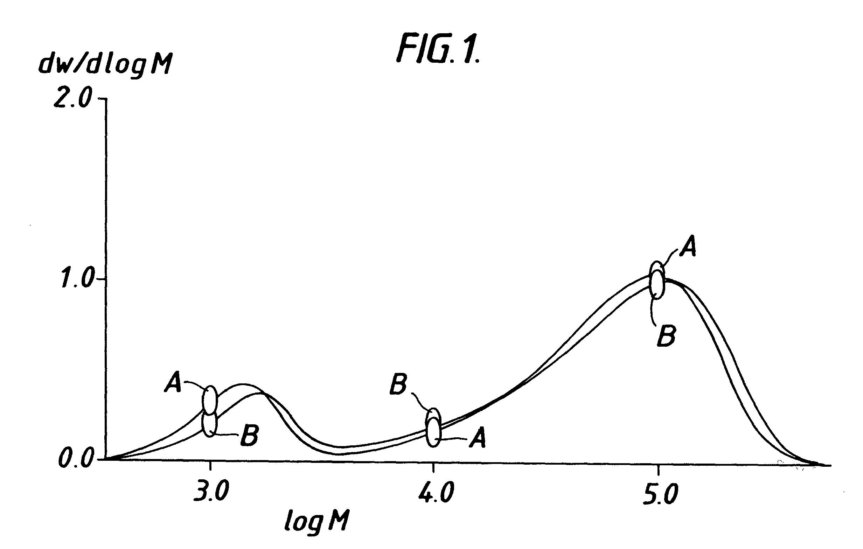

The results of the GPC analysis are illustrated in Figure 1.

Example 7

Coagulant Content

The calcium content of polyurethane films was determined after ashing the samples of the polyurethane film, taking up the ash in water (25ml) and then diluting further as appropriate. Calcium standards at 1,0, 2.0, 3.0 and 4.0 µg/ml in water were used to quantify the calcium present.
The results are shown in Table III:

Table III

| Polyurethane Sample | Batch No. | Calcium Content (mg/g polymer) | Mean (mg/g) |
|---|---|---|---|
| | | | |
| Unleached 40% coag | PP199205 | 7.95 | |
| | | 12.57 | 10.3 |
| Leached 40% coag | PP199206 | 9.03 | |
| | | 4.33 | 6.7 |
| Unleached 20% coag | PP199203 | 3.68 | |
| | | 3.92 | 3.8 |
| Leached 20% coag | PP199204 | 3.45 | |
| | | 2.45 | 3.0 |

Example 8

Surfactant Content

The method used to determine the amounts of surfactant (nonyl phenol ethoxylate) present in batches of WITCO BOND 740 emulsion involved soxhlet extraction with methanol of the films produced after drying. Followed by HPLC analysis on the extracts produced.
Preparation of polyurethane film by evaporating to dryness of the emulsion at 105°C followed by soxhlet extraction of 0.7-0.8g portions of the film with methanol (ca.100ml) for 4 hours. Extracts were quantitatively transferred into 100ml volumetric flasks and subsequently analysed by HPLC alongside nonyl phenol ethoxylate (surfactant) standards.
The results are shown in Tables IV and V:

Table IV

| Batch No. | % w/w Surfactant in Film | Mean | In emulsion |
|---|---|---|---|
| | | | |
| PPM039401 | 6.37 | | |

Table IV   (continued)

| Batch No. | % w/w Surfactant in Film | Mean | In emulsion |
|---|---|---|---|
|  | 7.00 | 6.69 | 4.01 |
| PPM119328 | 5.93 |  |  |
|  | 6.15 | 6.04 | 3.62 |
| PPM019433 | 6.72 |  |  |
|  | 6.79 | 6.76 | 4.06 |
| PPM039421 | 6.71 |  |  |
|  | 7.08 | 6.90 | 4.14 |
| PPM049428 | 7.85 |  |  |
|  | 7.96 | 7.90 | 4.74 |
| PPM069411 | 8.84 |  |  |
|  | 8.77 | 8.81 | 5.29 |

Table V

| Polyurethane Sample | % w/w Surfactant in Film |
|---|---|
|  |  |
| Unleached | 6.7 |
| Leached | 6.1 |

Example 9

Particle Size Determination

a) Malvern Analyser

The malvern particle size analyser is a light scattering based particle size analyser. It uses a 2 milliwatt Helium/Neon laser and a Fourier transform lens system to focus the scattered laser light on to a photosensitive silicon detector. The lens used was a 45mm lens which is used to measure particle diameters in the range of 0.1 to 80μm. The sample diluent was deionised water that had been filtered through a 0.2μm filter. The diluent particle size is measured prior to sample addition and is accounted for in the results as the background reading. The sample dilution was one drop from the end of a microspatula per 500ml of diluent.

The results are expressed in Table VI:

D [4,3]       Mean Diameter / Volume distribution
D [3,2]       Sauter Mean Diameter = Volume / Surface Area
D [v0.9]     Diameter at 90% volume
D [v0.1]     Diameter at 10% volume
D [v0.5]     Median Diameter

Table VI

| Batch No. | Particle Size (Microns) | | | | |
|---|---|---|---|---|---|
|  | D (4,3) | D (3,2) | Dv (0,9) | Dv (0,1) | Dv (0,5) |
| PPM 069332 | 3.81 | 0.9 | 8.87 | 0.4 | 2.54 |
| PPM 089322 | 2.56 | 1.07 | 5.17 | 0.54 | 2.12 |
| # 37144 | 2.09 | 0.93 | 4.17 | 0.46 | 1.76 |

Table VI   (continued)

| Batch No. | Particle Size (Microns) | | | | |
|---|---|---|---|---|---|
| | D (4,3) | D (3,2) | Dv (0,9) | Dv (0,1) | Dv (0,5) |
| PPM 119328 | 2 | 0.69 | 4.34 | 0.31 | 1.51 |
| PPM 119329 | 2.26 | 0.78 | 4.87 | 0.36 | 1.72 |
| PPM 019439 | 1.49 | 0.39 | 3.61 | 0.14 | 0.88 |
| PPM 039401 | 1.46 | 0.59 | 3.06 | 0.27 | 1.15 |
| PPM 039421 | 2.12 | 0.65 | 4.79 | 0.28 | 1.5 |
| PPM 049428 | 2.47 | 0.97 | 5.11 | 0.47 | 1.99 |
| PPM 059406 | 1.83 | 0.67 | 3.92 | 0.31 | 1.4 |
| PPM 0694011 | 1.39 | 0.49 | 3.07 | 0.21 | 1.01 |
| PPM 079409 | 2.61 | 1.46 | 4.76 | 0.79 | 2.37 |

b) Electron Microscopy

Samples were examined under an electron microscope at time 0 and time 2 minutes. The diameter of a substantial number of the particles was measured and the results are shown in Table VII.

Table VII

| Emulsion | Particle Size Time Zero | Particle Size on Coagulation (max time point 2 mins) |
|---|---|---|
| WITCOBOND 210-70 | 0.4µm -1.7µm | 0.4µm - 2.7µm* |
| | | |
| WITCOBOND 740 | 0.7µm - 3.3µm** | 0.7µm - 2.8µm** |

* Taken at two minute time point

** These results are diameter only

## Claims

1. A method of manufacturing a thin-walled polyurethane article having a wall thickness in the range from 0.03 mm to 1.0 mm, the method comprising dipping a shaped former into an aqueous polyurethane emulsion comprising at least 50% w/w of particles with a mean particle size of greater than 1.0 µm, as measured e.g. by electron microscopy, in combination with a plasticiser.

2. The method according to claim 1 wherein the emulsion comprises at least 50% w/w of particles with a mean particle size of from 1.0 µm to 2.5 µm.

3. The method according to claim 1 wherein the emulsion comprises at least 70% w/w of particles with a mean particle size of greater than 1.0 µm.

4. The method according to any one of the preceding claims wherein the amount of plasticiser in the polyurethane emulsion is from 0.1 to 40% w/w, preferably from 0.1 to 20% w/w, more preferably from 0.1 to 10% w/w, and most preferably from 5 to 10% w/w.

5. The method according to any one of the preceding claims wherein the plasticiser comprises a dipropylene glycol ester, a polyadipate, such as a polyneopentyl glycol adipate, or a diacid ester, preferably an alkanedioic acid ester and more preferably a dibenzoic acid ester.

6. The method according to any one of the preceding claims wherein the polyurethane emulsion contains from 0.1 to 10% w/w of a surfactant.

7. The method according to claim 6 wherein the surfactant is non-ionic.

8. The method according to any one of the preceding claims wherein the concentration of the polyurethane in the aqueous emulsion is from 10 to 60% w/w.

9. The method according to any one of the preceding claims wherein the polyurethane has a weight average molecular weight of from 50,000 to 1,000,000.

10. The method according to any one of the preceding claims which comprises the additional step of first dipping the former into a coagulant solution and optionally drying the coagulant coated former.

11. The method according to any one of the preceding claims wherein the polyurethane is the reaction product of an aliphatic diisocyanate and an aliphatic polyester.

12. The method according to any one of the claims 1 to 10 wherein the polyurethane is prepared from cyclohexane diisocyanate and alkylcyclohexane diisocyanate.

13. A thin-walled polyurethane article prepared according to the method of claim 1, comprising from 0.1 to 10% w/w of a surfactant in the polyurethane.

14. A thin-walled polyurethane article prepared according to the method of claim 1, wherein the polyurethane comprises from 2 to 15 milligrams of a coagulant residue per gram of polyurethane.

15. A thin-walled polyurethane article prepared according to the method of claim 1, wherein the polyurethane comprises from 0.1 to 40% w/w of a plasticiser.

16. A thin-walled polyurethane article prepared according to the method of claim 1, wherein the polyurethane has a weight average molecular weight of from 50,000 to 1,000,000.

17. A thin-walled polyurethane article prepared according to the method of claim 1, wherein the polyurethane is prepared from cyclohexane diisocyanate and alkylcyclohexane diisocyanate.

18. A thin-walled polyurethane article prepared according to the method of claim 1 wherein the article is a glove.

19. A thin-walled polyurethane article prepared according to the method of claim 1 wherein the article is a condom.

20. The use of an aqueous polyurethane emulsion containing at least 50% w/w of particles with a mean particle size of greater than 1.0 μm, as measured e.g. by electron microscopy, in combination with a plasticiser, in the manufacture of a thin-walled polyurethane article.

**Patentansprüche**

1. Verfahren zur Herstellung eines dünnwandigen Polyurethan-Artikels mit einer Wanddicke im Bereich von 0,03 mm bis 1,0 mm, unter Eintauchen eines profilierten Formers in eine wässerige Polyurethan-Emulsion mit zumindest 50 Gew.-% Partikel mit einer Partikelgröße von mehr als 1,0 μm, gemessen mittels Elektronenmikroskopie, in Verbindung mit einem Weichmacher.

2. Verfahren nach Anspruch 1, worin die Emulsion mindestens 50 Gew.-% Partikel mit einer durchschnittlichen Partikelgröße von 1,0 μm bis 2,5 μm aufweist.

3. Verfahren nach Anspruch 1, worin die Emulsion zumindest 70 Gew.-% Partikel mit einer durchschnittlichen Partikelgröße von mehr als 1,0 μm aufweist.

4. Verfahren nach einem der vorhergehenden Ansprüche, worin die Menge an Weichmacher in der Polyurethan-Emulsion zwischen 0,1 und 40 Gew.-%, vorzugsweise zwischen 0,1 und 20 Gew.-%, insbesondere zwischen 0,1 und 10 Gew.-% und am zweckmäßigsten zwischen 5 und 10 Gew.-% beträgt.

**5.** Verfahren nach einem der vorhergehenden Ansprüche, worin der Weichmacher einen Dipropylen-Glykolester, ein Polyadipat, wie zum Beispiel ein Polyneopentyl-Glykoladipat, oder einen Ester einer zweiwertigen Säure, vorzugsweise einen Dicarbonsäureester und am zweckmäßigsten einen Dibenzoesäureester umfaßt.

**6.** Verfahren nach einem der vorhergehenden Ansprüche, worin die Polyurethan-Emulsion zwischen 0,1 und 10 Gew.-% eines oberflächenaktiven Stoffs aufweist.

**7.** Verfahren nach Anspruch 6, worin der oberflächenaktive Stoff nichtionisch ist.

**8.** Verfahren nach einem der vorhergehenden Ansprüche, worin die Konzentration des Polyurethans in der wässerigen Emulsion zwischen 10 und 60 Gew.-% beträgt.

**9.** Verfahren nach einem der vorhergehenden Ansprüche, worin das Polyurethan ein gewichtsmittleres Molekulargewicht von 50.000 bis 1.000.000 besitzt.

**10.** Verfahren nach einem der vorhergehenden Ansprüche mit dem zusätzlichen Schritt, zunächst den Former in eine Koaguliermittellösung einzutauchen und gewünschtenfalls den koaguliermittelbeschichteten Former zu trocknen.

**11.** Verfahren nach einem der vorhergehenden Ansprüche, worin das Polyurethan das Reaktionsprodukt eines aliphatischen Diisocyanats und eines aliphatischen Polyesters ist.

**12.** Verfahren nach einem der Ansprüche 1 bis 10, worin das Polyurethan aus Cyclohexan-Diisocyanat und Alkylcyclohexan-Diisocyanat bereitet wird.

**13.** Dünnwandiger Polyurethan-Artikel, hergestellt gemäß Verfahren nach Anspruch 1, mit 0,1 bis 10 Gew.-% eines oberflächenaktiven Stoffs in dem Polyurethen.

**14.** Dünnwandiger Polyurethan-Artikel, hergestellt gemäß Verfahren nach Anspruch 1, worin das Polyurethan zwischen 2 und 15 mg eines Koaguliermittelrückstands pro Gramm Polyurethan enthält.

**15.** Dünnwandiger Polyurethan-Artikel, hergestellt gemäß Verfahren nach Anspruch 1, worin das Polyurethan zwischen 0,1 und 40 Gew.-% eines Weichmachers enthält.

**16.** Dünnwandiger Polyurethan-Artikel, hergestellt gemäß Verfahren nach Anspruch 1, worin das Polyurethan ein gewichtsmittleres Molekulargewicht zwischen 50.000 und 1.000.000 besitzt.

**17.** Dünnwandiger Polyurethan-Artikel, hergestellt gemäß Verfahren nach Anspruch 1, worin das Polyurethan aus Cyclohexan-Diisocyanat und Alkylcyclohexan-Diisocyanat hergestellt ist.

**18.** Dünnwandiger Polyurethan-Artikel, hergestellt gemäß Verfahren nach Anspruch 1, worin der Artikel ein Handschuh ist.

**19.** Dünnwandiger Polyurethan-Artikel, hergestellt gemäß Verfahren nach Anspruch 1, worin der Artikel ein Kondom ist.

**20.** Verwendung einer wässerigen Polyurethan-Emulsion mit zumindest 50 Gew.-% Partikeln einer Partikelgröße von mehr als 1,0 µm, gemessen mittels Elektronenmikroskopie, in Verbindung mit einem Weichmacher bei der Herstellung eines dünnwandigen Polyurethan-Artikels.

**Revendications**

**1.** Procédé de fabrication d'un article en polyuréthane à paroi mince ayant une épaisseur de paroi située dans la plage allant de 0,03 mm à 1,0 mm, le procédé comprenant l'immersion d'un calibre façonné dans une émulsion aqueuse de polyuréthane comprenant au moins 50 % en poids de particules ayant une granulométrie moyenne supérieure à 1,0 µm, telle que mesurée par exemple par microscopie électronique, en combinaison avec un plastifiant.

**2.** Procédé selon la revendication 1,

**caractérisé en ce que** l'émulsion comprend au moins 50 % en poids de particules ayant une granulométrie moyenne de 1,0 μm à 2,5 μm.

**3.** Procédé selon la revendication 1,

**caractérisé en ce que** l'émulsion comprend au moins 70 % en poids de particules ayant une granulométrie moyenne supérieure à 1,0 μm.

**4.** Procédé selon l'une quelconque des revendications précédentes,

**caractérisé en ce que** la quantité de plastifiant dans l'émulsion de polyuréthane est de 0,1 à 40 % en poids, de préférence de 0,1 à 20 % en poids, mieux encore de 0,1 à 10 % en poids, et tout spécialement de 5 à 10 % en poids.

**5.** Procédé selon l'une quelconque des revendications précédentes,

**caractérisé en ce que** le plastifiant comprend un ester de dipropylèneglycol, un polyadipate, tel qu'un poly (adipate de néopentylglycol), ou un ester de diacide, de préférence un ester d'acide alcanedioïque et mieux encore un ester d'acide dibenzoïque.

**6.** Procédé selon l'une quelconque des revendications précédentes,

**caractérisé en ce que** l'émulsion de polyuréthane contient de 0,1 à 10 % en poids d'un tensioactif.

**7.** Procédé selon la revendication 6,
**caractérisé en ce que** le tensioactif est non-ionique.

**8.** Procédé selon l'une quelconque des revendications précédentes,

**caractérisé en ce que** la concentration du polyuréthane dans l'émulsion aqueuse est de 10 à 60 % en poids.

**9.** Procédé selon l'une quelconque des revendications précédentes,

**caractérisé en ce que** le polyuréthane a une masse moléculaire moyenne en masse de 50 000 à 1 000 000.

**10.** Procédé selon l'une quelconque des revendications précédentes,

**caractérisé en ce qu'**il comprend l'étape additionnelle consistant à d'abord immerger le calibre dans une solution de coagulant et éventuellement à sécher le calibre revêtu de coagulant.

**11.** Procédé selon l'une quelconque des revendications précédentes,

**caractérisé en ce que** le polyuréthane est le produit de la réaction d'un diisocyanate aliphatique et d'un polyester aliphatique.

**12.** Procédé selon l'une quelconque des revendications 1 à 10,

**caractérisé en ce que** le polyuréthane est préparé à partir de diisocyanate de cyclohexane et de diisocyanate d'alkylcyclohexane.

**13.** Article en polyuréthane à paroi mince préparé conformément au procédé de la revendication 1,

**caractérisé en ce qu** 'il comprend de 0,1 à 10 % en poids d'un tensioactif dans le polyuréthane.

**14.** Article en polyuréthane à paroi mince préparé conformément au procédé de la revendication 1,

**caractérisé en ce que** le polyuréthane comprend de 2 à 15 milligrammes d'un résidu de coagulant par gramme de polyuréthane.

**15.** Article en polyuréthane à paroi mince préparé conformément au procédé de la revendication 1,

**caractérisé en ce que** le polyuréthane comprend de 0,1 à 40 % en poids d'un plastifiant.

**16.** Article en polyuréthane à paroi mince préparé conformément au procédé de la revendication 1,

**caractérisé en ce que** le polyuréthane a une masse moléculaire moyenne en masse de 50 000 à 1 000 000.

**17.** Article en polyuréthane à paroi mince préparé conformément au procédé de la revendication 1,

**caractérisé en ce que** le polyuréthane est préparé à partir de diisocyanate de cyclohexane et de diisocyanate d'alkylcyclohexane.

**18.** Article en polyuréthane à paroi mince préparé conformément au procédé de la revendication 1, **caractérisé en ce que** l'article est un gant.

**19.** Article en polyuréthane à paroi mince préparé conformément au procédé de la revendication 1, **caractérisé en ce que** l'article est un préservatif.

**20.** Utilisation d'une émulsion aqueuse de polyuréthane **caractérisée en ce qu'**elle contient au moins 50 % en poids de particules ayant une granulométrie moyenne supérieure à 1,0 $\mu$m, telle que mesurée par exemple par microscopie électronique, en combinaison avec un plastifiant, dans la fabrication d'un article en polyuréthane à paroi mince.

FIG.1.